# EUROPÄISCHE PATENTANMELDUNG

(11) **EP 0 028 363 A1**
(43) Veröffentlichungstag der Anmeldung: **13.05.1981**
(21) Anmeldenummer: 80106461.9
(22) Anmeldetag: 23.10.1980
(51) Int. Cl.: C07D 249/08, A01N 43/64, A01N 55/00, C07F 7/18

(54) **Entriazole, ihre Herstellung, ihre Verwendung zur Bekämpfung von Fungi und Mittel dafür**

(30) Priorität: 02.11.1979 DE 2944223
(71) Anmelder: BASF Aktiengesellschaft, 67063 Ludwigshafen (DE)
(72) Erfinder: Zeeh, Bernd, Dr., D-6700 Ludwigshafen (DE); Buschmann, Ernst, Dr., D-6700 Ludwigshafen (DE); Pommer, Ernst-Heinrich, Dr., D-6703 Limburgerhof (DE)

(57) **Zusammenfassung**

Die vorliegende Erfindung betrifft Entriazole der Formel 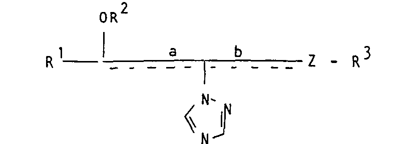 in der
R¹ tert.-Butyl oder einen Phenylrest, der durch ein oder mehrere Halogenatome substituiert sein kann,
R² einen Alkyl-, Alkenyl-, Alkinylrest mit bis zu 5 C-Atomen, einen gegebenenfalls durch ein oder mehrere Halogenatome substituierten Aralkylrest oder einen C₁₋₄-Trialkylsilylrest
R' einen gegebenenfalls durch ein oder mehrere Halogenatome oder durch Phenyl substituierten Arylrest darstellen und
Z CH₂, CH oder 0 bedeutet, wobei die Doppelbindung für Z=CH₂ und 0 die Position a und fürZ = CH die Position beinnimmt,

sowie deren Säureadditionssalze und Metallkomplexe.

Weiter betrifft die Erfindung die Herstellung dieser Entriazole, deren Verwendung bei der Bekämpfung von Fungi und Fungizide, die diese Entriazole enthalten.

## Beschreibung

Die vorliegende Erfindung betrifft neue Entriazole, Verfahren zu ihrer Herstellung, deren Verwendung als Fungizide, fungizide Mischungen, die diese Wirkstoffe enthalten, Verfahren zur Herstellung solcher fungizider Mischungen sowie Verfahren zur Bekämpfung von Schadpilzen mit diesen Fungiziden oder fungiziden Mischungen, die diese Verbindungen enthalten.

Eine große Anzahl fungizider Triazolverbindungen ist bekannt, vgl. DE-OS 25 56 319, 26 34 511, 27 23 942, 27 24 684, 27 35 314, 27 38 725, 27 45 827. Wenig bekannt ist über Entriazole, d.h. Triazole, die am Stickstoff eine Vinylgruppe besitzen.

Enamine sind als leicht hydrolysierbare Verbindungen bekannt (E.J. Stamhuis in "Enamines", Marcel Dekker, 1969 New York, Seite 101). Enamine sind deshalb als Wirkstoffe für den Pflanzenschutz nicht geeignet. Da Entriazole auch der Substanzklasse der Enamine zuzurechnen sind, überrascht es nicht, daß nur wenig über Entriazole und ihre Verwendbarkeit bekannt ist. Die bislang beschriebenen Entriazole (DE-OS 26 45 617 und 27 57 113) sind in ihrer Wirkung als Pflanzenschutzmittel unzureichend.

Es wurde nun gefunden, daß Entriazole der Formel 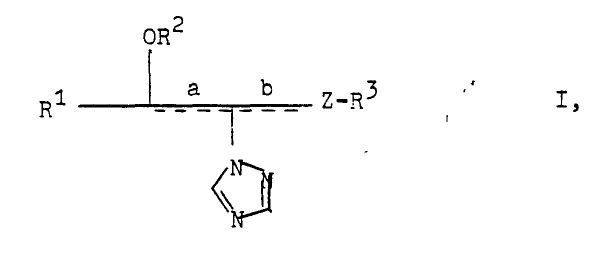 In der
_{R}¹ tert.-Butyl oder einen Phenylrest, der durch ein oder mehrere Halogenatome substituiert sein kann,
_{R}² einen Alkyl-, Alkenyl-, Alkinylrest mit bis zu 5 C-Atomen, einen gegebenenfalls durch ein oder mehrere Halogenatome substituierten Aralkylrest oder einen C₁₋₄-Trialkylsilylrest
_{R}³ einen gegebenenfalls durch ein oder mehrere. Halogenatome oder durch Phenyl substituierten Arylrest darstellen und
Z CH₂, CH oder 0 bedeutet, wobei die Doppelbindung für Z=CH₂ und 0 die Position a und für Z = CH die Position b einnimmt,

sowie deren Säureadditionssalze und Metallkomplexe eine erstaunlich gute Wirksamkeit als Fungizide besitzen.

In der Formel I bedeuten R¹ vorzugsweise tert.-Butyl, Phenyl, 2-Chlorphenyl, 3-Chlorphenyl, 4-Chlorphenyl, 2,4-Dichlorphenyl, 4-Fluorphenyl, 4-Bromphenyl; _{R}² besonders Methyl, Ethyl, Allyl, Propinyl, Benzyl, 4-Chlorbenzyl, 2,4-Dichlorbenzyl, 4-Brombenzyl und R³ vorzugsweise Phenyl, 2-Chlorphenyl, 4-Chlorphenyl, 2,4-Dichlorphenyl, 4-Fluorphenyl, 4-Bromphenyl.

Es wurde weiterhin gefunden, daß man die neuen Entriazole der Formel I dadurch herstellen kann, daß man
a) ein Triazolylketon der Formel IV 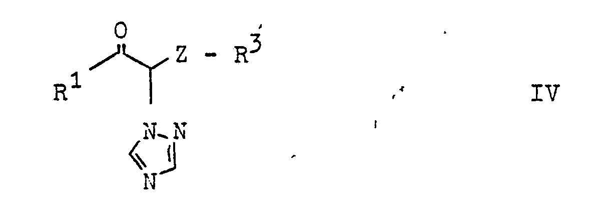 worin _{R}¹, R³ und Z die angegebene Bedeutung haben oder
b) - falls die Doppelbindung in Position b ist und Z ein Kohlenstoffatom bedeutet - eine Verbindung der Formel V 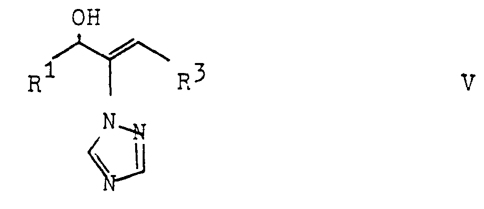 worin R^{l} und R³ die angegebene Bedeutung haben, mit einer Verbindung der Formel VI 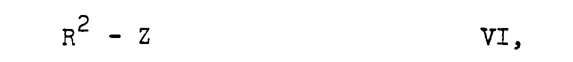 worin R² die angegebene Bedeutung hat und Z eine nucleofuge Abgangsgruppe darstellt, in Gegenwart eines Säurefängers umsetzt und die so erhaltenen Verbindungen gegebenenfalls in ihre Säureadditionssalze oder Metallkomplexe überführt.

Die Alkylierung von Triazolylketonen ist aus der DE-OS 26 10 022 bekannt. Isoliert wurden dabei nur Kohlenstoff-alkylierte Produkte. Die Triazolylketone der Formel II werden nun überraschenderweise am Sauerstoff alkyliert. So erhält man bei der Alkylierung der Triazolylketone IVa und IVb unter geeigneten Bedingungen die erfindungsgemäßen Entriazole IVc-IVc:

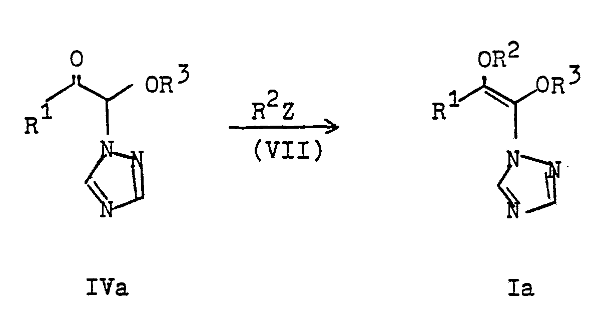

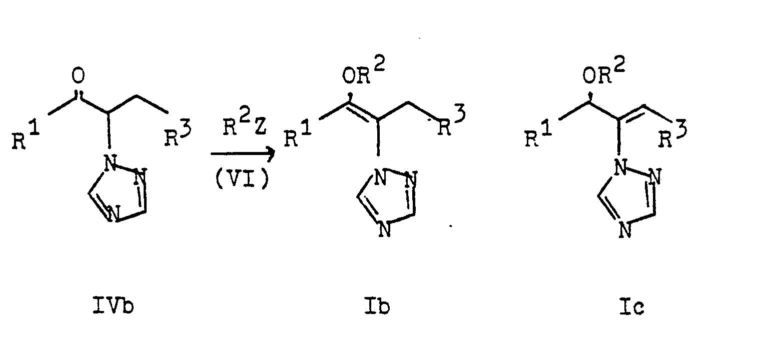

Bei der Alkylierung der Triazolylketone können durch Verschiebung der Doppelbindung die Isomere (vgl. Ib und Ic) entstehen, die jeweils wieder in je zwei verschiedenen isomeren.Formen vorliegen können.

Die Ketone IV und die Allylalkohole V werden mit den Alkylierungsmitteln VII in einem geeigneten Lösungsmittel in Gegenwart eines Säurefängers umgesetzt. Ketone der Formel II sind beispielsweise die in Tabelle 1 aufgeführten Verbindungen.

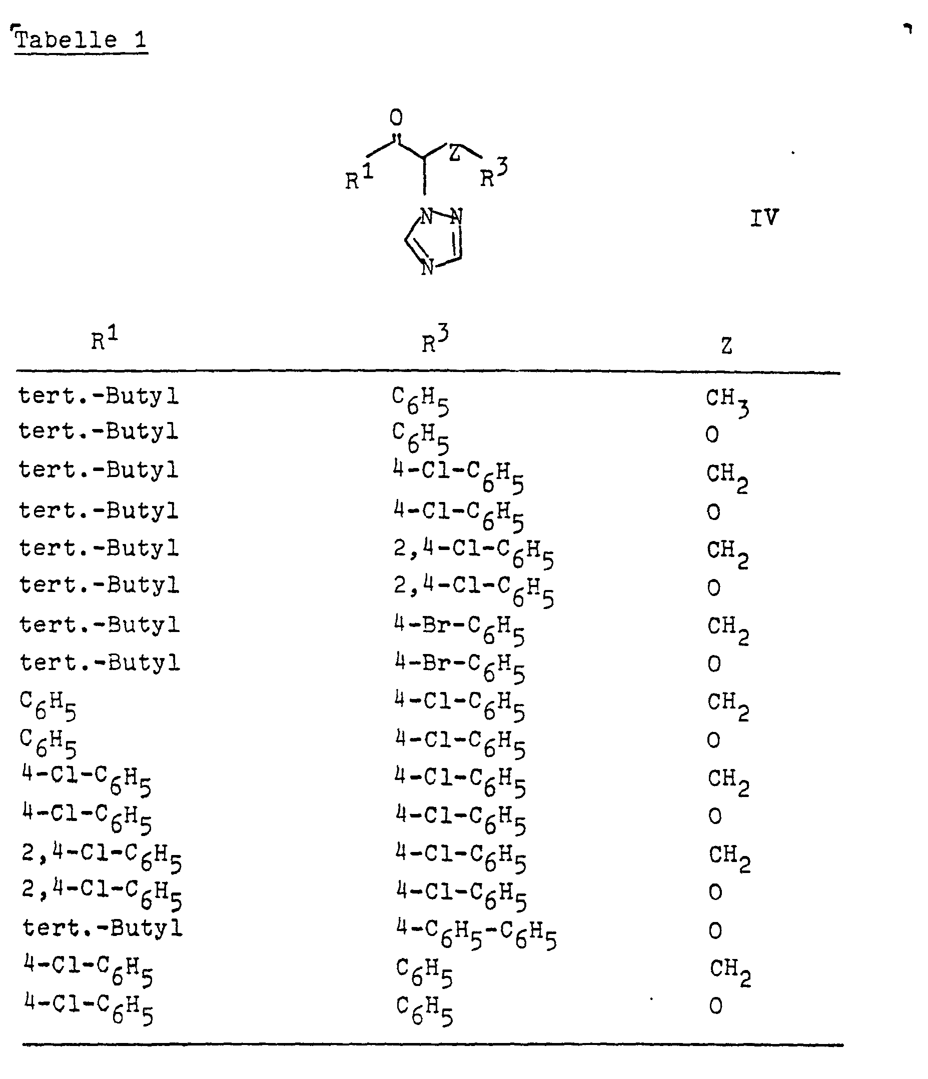

Verbindungen der Formel VI sind beispielsweise: Methyliodid, Methylbromid, Dimethylsulfat, Ethyliodid, Ethylbromid, Diethylsulfat, Allylbromid, Diallylsulfat, Propargylbromid, Crotonylbromid, n-Propylbromid, n-Propylsulfat, Benzylchlorid, Benzylbromid, 4-Chlorbenzylchlorid, 4-Chlorbenzylbromid, 2,4-Dichlorbenzylchlorid, 2,4-Dichlorbenzylbromid, 4-Fluorbenzylchlorid, 4-Fluorbenzylbromid, Trimethylchlorsilan, Triethylchlorsilan, N-Trimethylsilylacetamid.

Geeignete Lösungsmittel sind beispielsweise: Dimethylacetamid, Dimethylformamid, Hexamethylphosphorsäuretriamid, Tetrahydrofuran, Dimethylsulfoxid, N-Methylpyrrolidon, Dioxan. Bevorzugt ist Dimethylformamid.

Als Säurefänger eignen sich Natrium- und Kaliumhydrid, Kaliumamid, Natriumhydrid, Lithium-diisopropylamid und Butyllithium.

Die geeignete Reaktionstemperatur liegt zwischen -40 und +50°C. Bevorzugter Temperaturbereich ist 0-20°C.

Falls gewünscht, werden die Entriazole der Formel I anschließend nach bekannten Verfahren in ihre für Pflanzen verträgliche Salze oder in ihre Metallkomplexe überführt.

Zur Salzbildung eignen sich beispielsweise: Salzsäure, Bromwasserstoffsäure, Schwefelsäure, Salpetersäure, Phosphorsäure, Essigsäure oder Dodecylbenzolsulfonsäure. Die Wirksamkeit der Salze geht auf das Kation zurück, so daß die Wahl des Anions beliebig ist.

Metallkomplexe bilden sich durch Anlagerung der neuen Verbindungen an die Kationen von Metallsalzen. Besonders eignen sich hierfür Kupfer(II)-chlorid, Kupfer(II)-sulfat, Kupfer(II)-nitrat, Zink(II)-chlorid, Eisen(III)-chlorid, Mangan(II)-chlorid, Nickel(II)-bromid.

Ausgangsverbindungen für die erfindungsgemäßen Entriazole der Formel I sind die teilweise aus-DE-OS 22 01 063 und DE-OS 26 38 470 bekannten Triazolketone der Formel IVa und IVb, die nach folgendem Schema nach allgemein bekann- r ten Reaktionen hergestellt werden können:

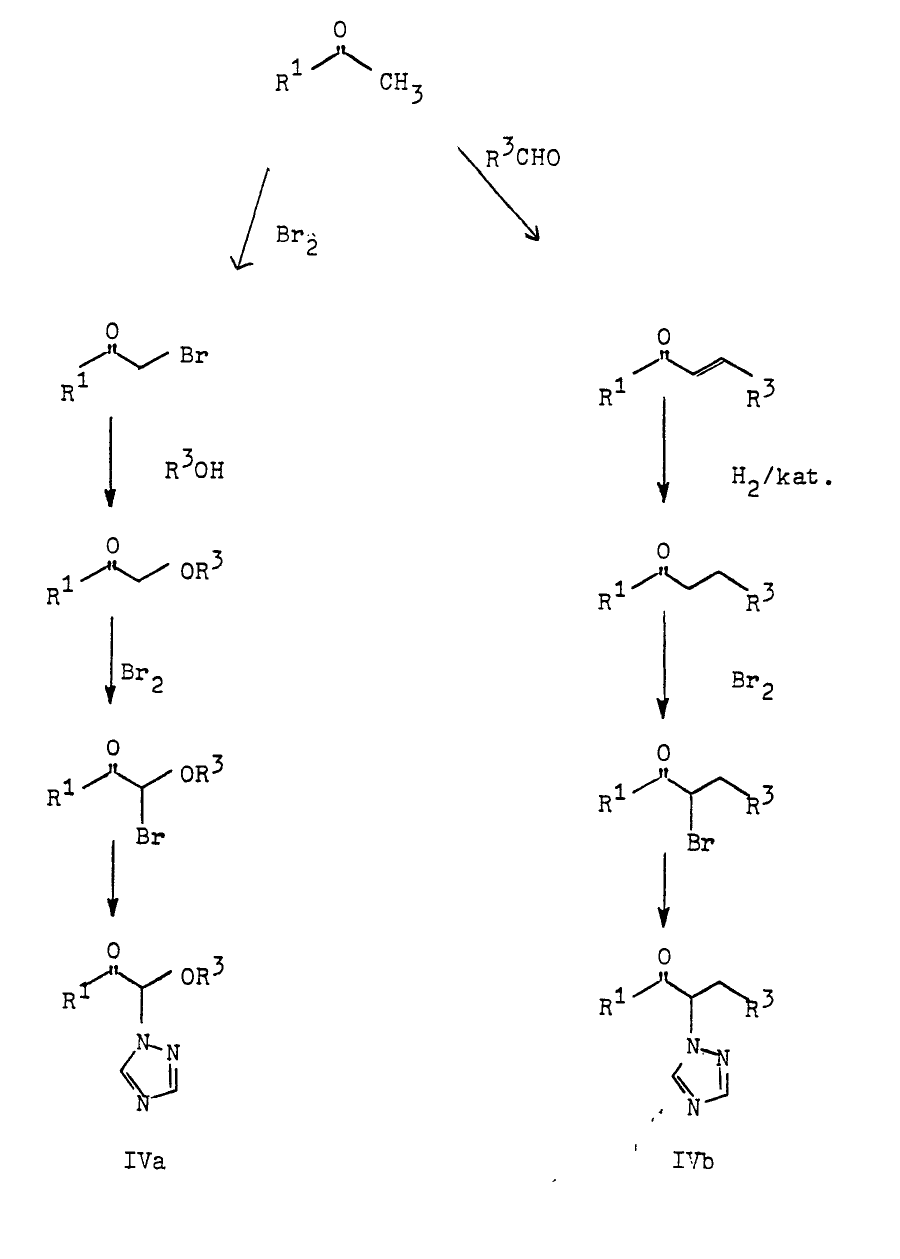

Geeignete Ausgangsverbindungen sind auch die aus der DE-OS 28 38 847 bekannten Triazolallylalkohole der Formel V.

Die folgenden Beispiele erläutern die Herstellung der neuen Verbindungen:

### Beispiel 1

Zu einer Suspension von 2,6 g Natriumhydrid in 80 ml wasserfreiem Dimethylformamid (DMF) wird bei 20°C eine Lösung von 29,4 g 1-(4-Chlorphenoxy)-3,3-dimethyl-1-(1,2,4--triazol-1-yl)-butan-2-on in 50 ml DMF zugetropft.

Nach 2 h Rühren bei Raumtemperatur kühlt man auf 5 bis 10°C und tropft eine Lösung von 20,6 g 4-Chlorbenzylbromid in 20 ml-DMF hinzu. Man rührt 5 h bei Raumtemperatur, tropft 300 ml H₂0 zu und extrahiert dreimal mit je 100 ml CH₂Cl₂. Die organische Phase wird mehrfach mit Wasser gewaschen, über Na₂SO₄ getrocknet und eingeengt. Das zurückbleibende Öl kristallisiert beim Verreiben mit Petrolether. Die Kristalle werden mit Petrolether gewaschen und im Vakuum getrocknet. Man erhält 4,5 g 2-(4-Chlorbenzyl- oxy)-1-(4-chlorphenoxy)-3,3-dimethyl-(1,2,4-triazol-1-yl)--1-buten (la), Fp. 130-132°C.

Aus obigem Petrolether-Filtrat kristallisieren nach 2 Tagen Stehen bei Raumtemperatur 2,5 g 2-(4-Chlorbenzyl- oxy)-1-(4-Chlorphenoxy)-3,3-dimethyl-(1,2,4-triazol-1-yl)--1-buten (1b), Fp. 105-107°C. 1b ist ein Stereoisomeres zu la.

### Beispiel 2

Zu einer Suspension von 2,6 g Natriumhydrid in 50 ml Dimethylformamid wird bei 20°C eine Lösung von 29,2 g 5-(4--Chlorphenyl)-2,2-dimethyl-4-(1,2,4-triazol-1-yl)-pentan--3-on in 80 ml Dimethylformamid zugetropft. Nach 2 h Rühren bei Raumtemperatur wird bei 5-10°C eine Lösung von 12,6 g Dimethylsulfat in 20 ml Dimethylformamid zugetropft. Man rührt 5 h bei Raumtemperatur, tropft 300 ml Wasser zu und extrahiert mit CH₂Cl₂. Die organische Phase wird mit Wasser gewaschen und über Na₂S0₄ getrocknet und eingeengt. Das zurückbleibende öl kristallisiert beim Verreiben mit Petrolether. Umkristallisation aus Essigester/Petrolether 3:5 ergibt 3,3 g 1-(4-Chlorphenyl)-3-methoxy-4,4-dimethyl--2-(1,2,4-triazol-1-yl)-2-penten, Fp. 157-159°C.

### Beispiel 3

Zu einer Suspension von 2,6 g Natriumhydrid in 50 ml Dimethylformamid wird bei 20°C eine Lösung von 32,6 g 5--(2,4-Dichlorphenyl)-2,2-dimethyl-4-(1,2,4-triazol-1-yl)--pentan-3-on in 90 ml Dimethylformamid zugetropft. Nach 2 h Rühren bei Raumtemperatur wird bei 5-10°C eine Lösung von 12,6 g Dimethylsulfat in 20 ml Dimethylformamid zu- getrop'ft. Man rührt 5 h bei Raumtemperatur, tropft 300 ml Wasser zu und extrahiert mit CH₂Cl₂. Die organische Phase wird mit Wasser gewaschen über Na₂S0₄ getrocknet und eingeengt. Chromatographie über Kieselgel (Petrolether/ Toluol) ergibt 3,3 g 1-(2,4-Dichlorphenyl)--3-methoxy-4,4-dimethyl-2-(1,2,4-triazol-1-yl)-1-penten, Fp. 87 bis 90°C.

### Beispiel 4

5,8 g 1-p-Chlorphenyl-4,4-dimethyl-2-(1,2,4-triazol-1-yl)--1-penten-3-ol (bekannt aus DE-OS 28 38 847) und 5,2 g N-Trimethylsilylacetamid werden in 100 ml wasserfreiem Toluol 5 h zum Rückfluß erhitzt. Man kühlt ab, wäscht die Toluollösung dreimal mit 50 ml Wasser, trocknet über Na₂SO₄ und engt ein. Zurück bleibt 1-p-Chlorphenyl-4,4-dimethyl--2-(1,2,^{h}-triazol-1-yl)-3-trimethylsiloxy-1-penten als gelbliches öl.

Analog wurden hergestellt:

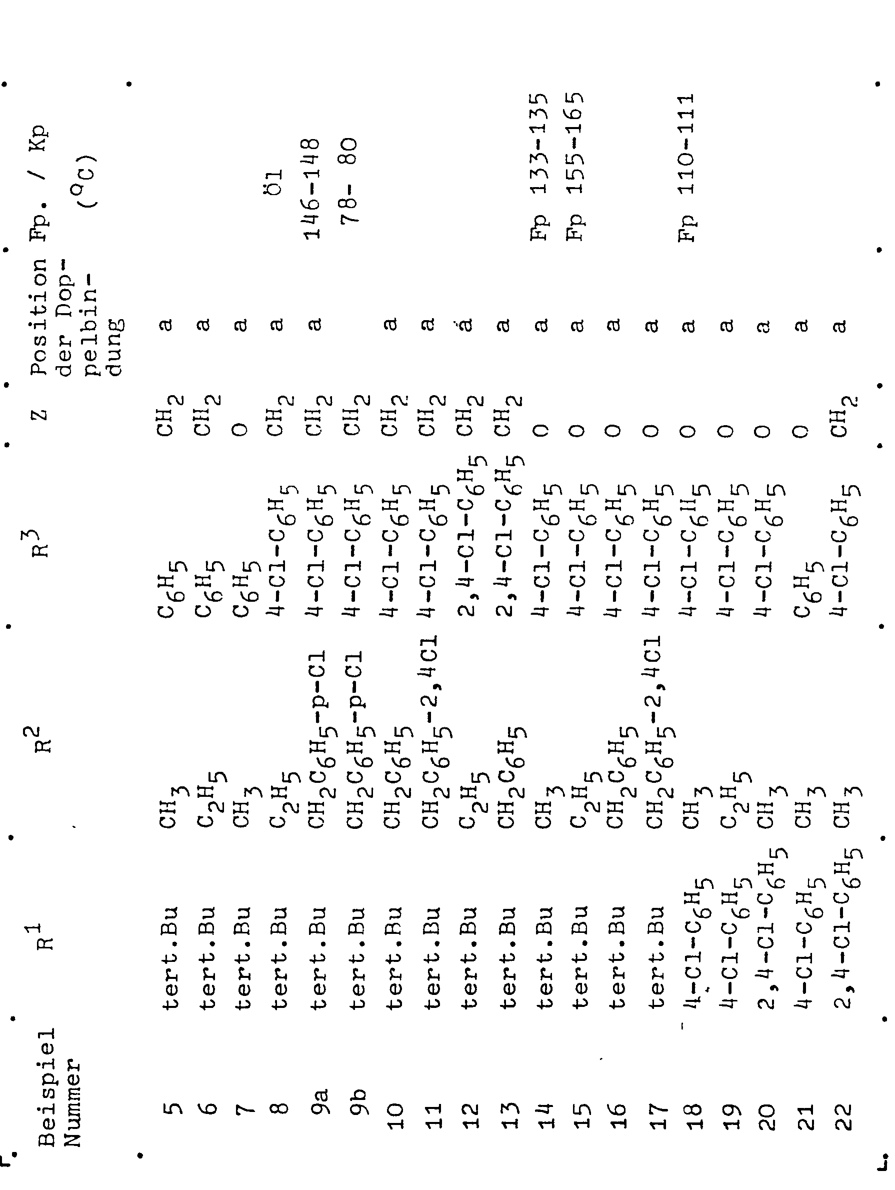

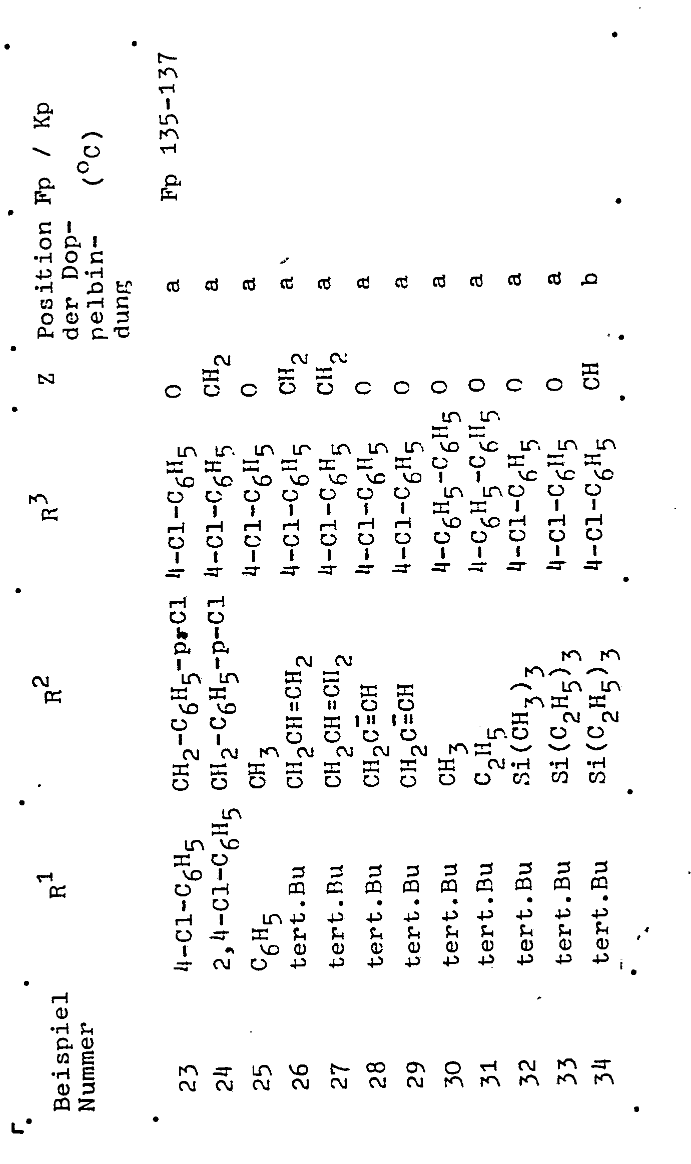

Die erfindungsgemäßen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz, u.a. zur Bekämpfung holzzerstörender Pilze, wie Coniophora puteana und Polystictus versicolor, verwendet werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoriacearum (echter Mehltau) an Kürbisgewächsen, Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie
Helminthosporiumarten an Getreide,
Ustilago-Arten an Getreide und Zuckerrohr,
Rhynchosporium secale an Getreide,
Venturia inaequalis (Apfelschorf). ,

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemäßen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdclfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen - Fettalkohol - Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha.

Die neuen Verbindungen können auch im Materialschutz u.a. zur Bekämpfung holzzerstörender Pilze, wie Coniophora puteana und Polystictus versicolor eingesetzt werden. Bei der Anwendung der Wirkstoffe im Materialschutz, z.B. als Fungizide für Anstrichfarben und Weich-Polyvinylchlorid, betragen die Aufwandmengen 0,05 bis 5 Gew.% Wirkstoff, bezogen auf das Gesamtgewicht der zu konservierenden Farben bzw. des mikrozidauszurüstenden Polyvinylchlorids. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:
I. Man vermischt 90 Gewichtsteile der Verbindung des Beispiels 1 mit 10 Gewichtsteilen N-Methyl-α-pyrrolidon und erhält eine Lösung, die zur Anwendung in Form kleinster Tropfen geeignet ist.
II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
III. 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile der Verbindung des Beispiels 5 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
V. 20 Gewichtsteile der Verbindung des Beispiels 6 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichts-teilen Wasser erhält man eine Spritzbrühe, die 0,1 Gew.% des Wirkstoffs enthält.
VI. 3 Gewichtsteile der Verbindung des Beispiels 1 werden mit 97 Gewichtsteilen feinteiligem Kaolin innig vermischt. Man erhält auf diese Weise ein Stäubemittel, das 3 Gew.% des Wirkstoffs enthält.
VII. 30 Gewichtsteile der Verbindung des Beispiels 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII.40 Gewichtsteile der Verbindung des Beispiels 3 werden mit 10 Teilen Natriumsalz eines Phenolsulfonsäure--harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.
IX. 20 Teile der Verbindung des Beispiels 4 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.
X. 1 Teil der Verbindung des Beispiels 13 wird mit 15 Teilen eines Alkydharzes mit mittlerem Gehalt an ölen (20 % Festharz) gemischt. Anschließend fügt man 45 Teile einer aromatenhaltigen Benzinfraktion hinzu, filtriert gegebenenfalls, um Verunreinigungen zu beseitigen und füllt mit einer aliphatenhaltigen Benzinfraktion auf 100 Teile auf.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel, Dithiocarbamate und deren Derivate, wie Ferridimethyldithiocarbamat, Zinkdimethyldithiocarbamat, Manganethylenbisdithiocarbamat, Mangan-Zink-ethylendiamin-bis-dithiocarbamat oder Zinkethylenbisdithiocarbamat, Tetramethylthiuramidsulfide, Ammoniak-Komplex von Zink-(N,N-ethylen-bis-dithiocarbamat) und N,N'-Polyethylen-bis-(thiocarbamoyl)-disulfid, Zink-(N,N'-propylen-bis-dithiocarbamat), Ammoniak-Komplex von Zink-(N,N'-propylen-bis-dithiocarbamat) und N,N'-Propylen-bis(thiocarbamoyl)-disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarb,onat;
heterocyclische Substanzen, wie N-Trichlormethylthio-tetrahydrophthalimid, N-(1,1,2,2-Tetrachlorethylthio)-tetrahydrophthalimid, N-Trichlormethylthio-phthalimid, 2-Heptadecyl-2-imidazolin-acetat, 2,4-Dichlor-6-(o-chloranilino)-s-triazin, 0,0-Diethyl-phthalimidophosphonothioat, 5-Amino-1-(bis-(dimethylamino)-phosphinyl)-3-phenyl-1,2,4--triazol, 5-Ethoxy-3-trichlormethyl-1,2,4-thiadiazol, 2,3-Dicyano-1,4-dithioanthrachinon, 2-Thio-l,3-dithio-(4,5-b)-chinoxalin, 1-(Butylcarbamoyl)-2-benzimidazol-carbaminsäuremethylester, 2-Methoxycarbonylamino-benzimidazol, 2-Rhodanmethylthio-benzthiazol, 4-(2-Chlorphenylhydrazono)-3-methyl-5-isoxazolon, Pyridin-2-thio-l-oxid, 8-Hydroxychinolin bzw. dessen Kupfersalz, 2,3-Dihydro-5-carboxanilido-6-methyl-1,4-oxathiin-4,4--dioxid, 2-(Furyl-(")-benzimidazol, Piperazin-1,4-diyl-bis-(1-(2,2,2-trichlor-ethyl)-formamid), 2-(Thiazolyl-(4))-benzimidazol, 5-Butyl-2-dimethylamino-4-hydroxy-6-methyl-pyrimidin, Bis-(p-Chlorphenyl)-pyridinmethanol, 1,2-Bis-(3-ethoxycarbonyl-2-thioureido)-benzol, 1,2-Bis-(3-methoxycarbonyl-2-thioureido)-benzol
und weitere Substanzen, wie Dodecylguanidinacetat, 3-(3-(3,5-Dimethyl-2-oxycyclohexyl)-2-hydroxyethyl)--glutarimid, Hexachlorbenzol, N-Dichlorfluormethylthio-N',N'-dimethyl-N-nhenyl-schwefel- säurediamid 2,5-Dimethyl-furan-3-carbonsäureanilid, 2,5-Dimethyl-furan-3-carbonsäure-cyclohexylamid,
2-Cyan-N-(ethylaminocarbonyl)-2-(methoxyimino)-acetamid, 2-Methyl-benzoesäure-anilid, 2-Iod-benzoesäure-anilid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, DL-Methyl-N-(2,6-dimethyl-phenyl)-N-furoyl(2)-alaninat, DL-N-(2,6-Dimethyl-phenyl)-N-(2'-methoxyacetyl-alanin--methylester, 5-Nitro-isophthalsäure-di-isopropylester, 1-(1',2',4'-Triazolyl-1')-(4'-chlorphenoxy)-3,3-dimethylbutan-2-on, 1-(1',2',4'-Triazolyl-1')-1-(4'-chlorphenoxy)-3,3-dimethylbutan-2-ol, N-(2,6-Dimethylphenyl)-N-chloracetyl-D,L-2-aminobutyro- lacton, N-(n-Propyl)-N-(2,4,6-trichlorphenoxyethyl)-N'-imidazolyl- harnstoff.

Die folgenden Beispiele A und B zeigen die biologische Wirkung der neuen Substanzen. Als Vergleichssubstanz diente in Beispiel A das aus der DE-OS 20 63 857 bekannte 1-(2'-(2",4"-Dichlorphenyl)-2'-(2"-propenyloxy)-ethyl-1H--imidazol (Y) und in Beispiel B die aus der DE-OS 26 45 617 bekannte Substanz (Z):

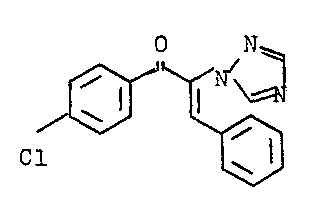

### Beispiel A

Blätter von in Töpfen gewachsenen Weizenkeimlingen der Sorte "Jubilar" werden mit wäßrigen Emulsionen aus 80 Ge- wichtsprozent Wirkstoff und 20 Gewichtsprozent Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltauentwicklung ermittelt.

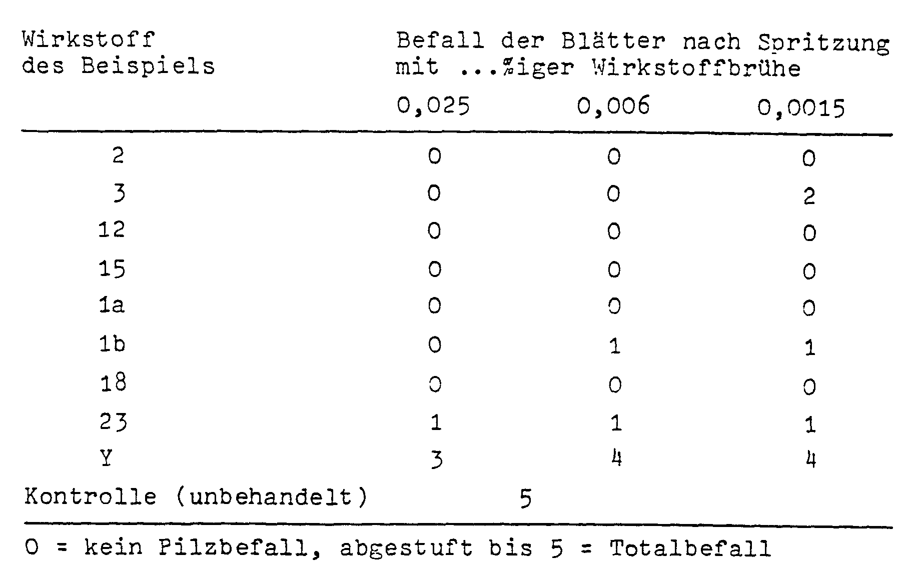

### Beispiel B

Blätter von in Töpfen gewachsenen Weizensämlingen der Sorte "Caribo" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit 0,025, 0,006 und 0,0015 %igen (Gew.-%) wäßrigen Spritzbrühen, die 80 % Wirkstoff und 20 % Ligninsulfonat in der Trockensubstanz enthalten, tropftnaß gespritzt. Nach dem Antrocknen des Spritzbelages werden die Versuchspflanzen im Gewächshaus bei Temperaturen zwischen 20 und 22°C und 65 bis 70 % relativer Luftfeuchte aufgestellt. Nach 8 Tagen wird das Ausmaß der Rostpilzentwicklung auf den Blättern ermittelt:

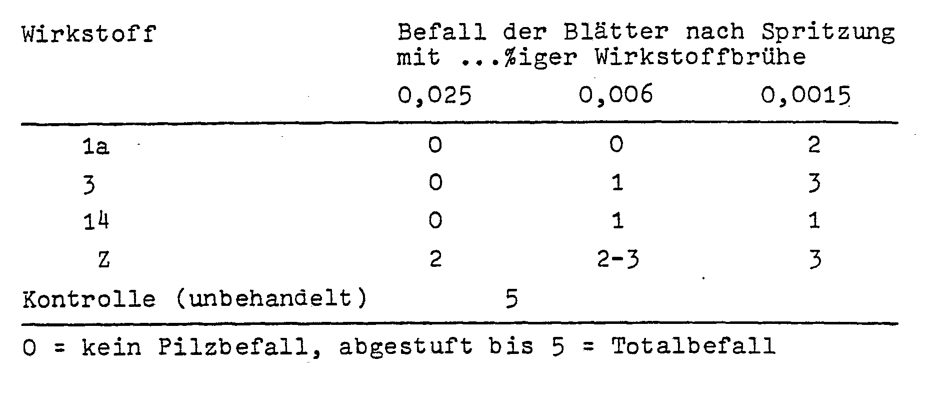

### Beispiel C

Zur Ermittlung der fungiziden Wirksamkeit gegenüber den holzzerstörenden Pilzen Coniophora puteana und Trametes versicölor urden in Anlehnung an die DIN-Vorschrift 52 176, Blatt 1, "Prüfung von Holzschutzmitteln, Mykologische Kurzprüfung (Klötzchenverfahren)", Kiefernsplintholzklötzchen mit den Abmessungen 50 x 25 x 15 mm mit jeweils 100 g/m² Holzoberfläche oliger Holzschuztmittelzubereitungen, die 1 % Wirkstoff enthielten, bestrichen. Nach vierwöchiger Lagerung wurden die behandelten Klötzchen zusammen mit den unbehandelten in Glasschalen gelegt, die als Prüfpilz Coniophora puteana bzw. Trametes versicolor auf einem Nähragar enthielten. Die Schalen wurden, anschließend in einem Klimaraum bei einer Temperatur von 22°C und einer relativer Luftfeuchte von 70 % bebrütet. Nach dreimonatiger Versuchs- dauer wurden die Klötzchen von anhaftendem Pilzmycel befreit und getrocknet. Anschließend wurde das Ausmaß der Holzzerstörung festgestellt.

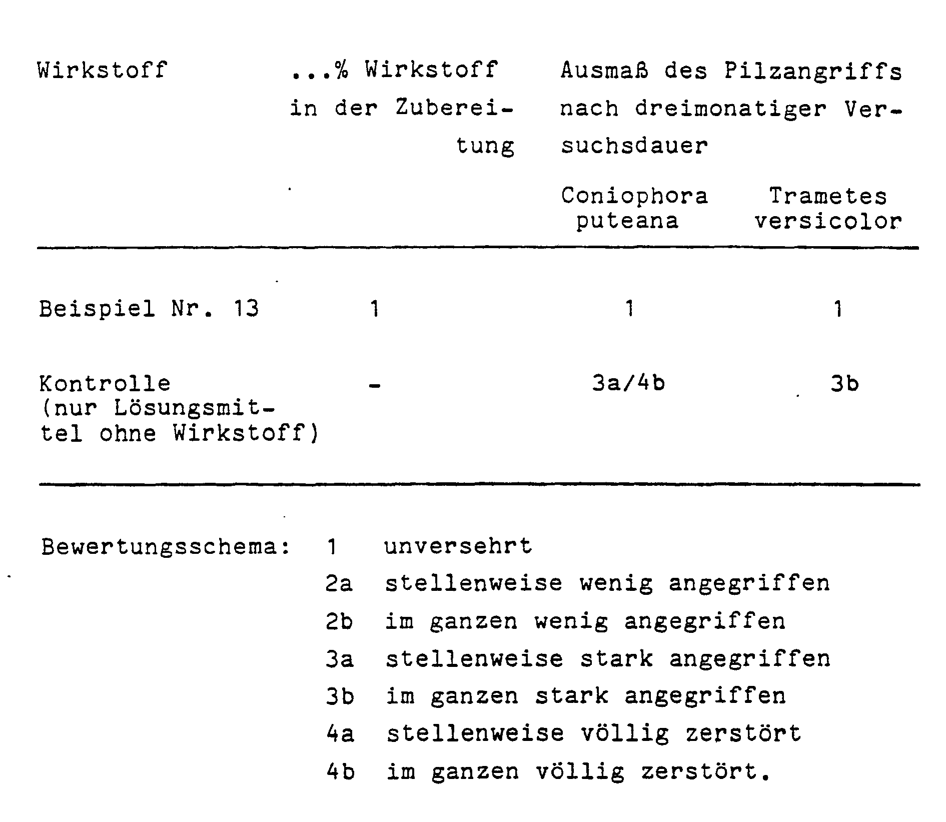

## Patentansprüche

1. Entriazole der Formel I 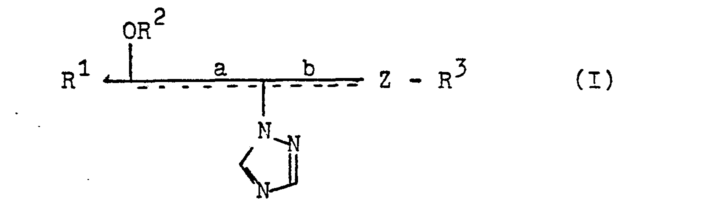 in der
R¹ tert.-Butyl oder einen Phenylrest, der durch ein oder mehrere Halogenatome substituiert sein kann,
R² einen Alkyl-, Alkenyl-, Alkinylrest mit bis zu 5 C-Atomen, einen gegebenenfalls durch ein oder mehrere Halogenatomen substituierten Aralkylrest oder einen C₁₋₄-Trialkylsilylrest
R einen gegebenenfalls durch ein oder mehrere Halogenatome oder durch Phenvl substituierten Arylrest darstellen und
Z CH₂, CH oder 0 bedeutet, wobei die Doppelbindung für Z=CH₂ und 0 die Position a und für Z = CH die Position b einnimmt,
sowie deren Säureadditionssalze und Metallkomplexe.

2. Entriazole gemäß Anspruch 1 der Formel II 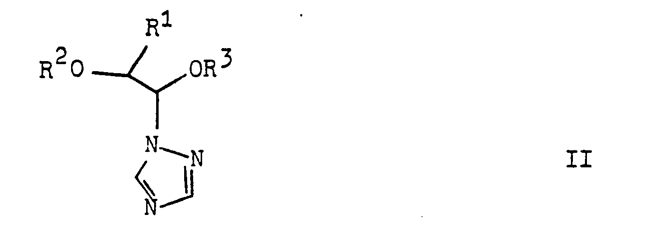 in der R^{1,} R² und R³ die im Anspruch 1 angegebenen Bedeutungen haben.

3. Entriazole gemäß Anspruch 1 der Formel III 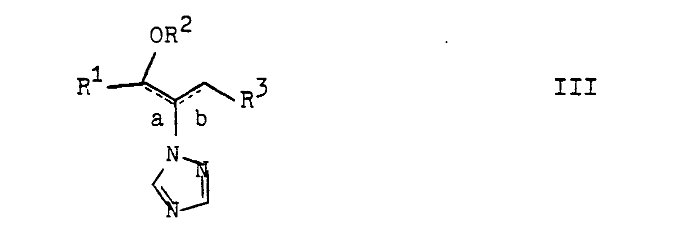 in der R¹, R² und R³ die im Anspruch 1 angegebenen Bedeutungen haben, wobei die Doppelbindung entweder die Position a oder b einnehmen kann.

4. Entriazol, ausgewählt aus der Gruppe bestehend aus 1-(4-Chlorphenoxy)-2-methoxy-3,3-dimethyl-1-(1,2,4--triazol-1-yl)-1-buten, 1-(4-Chlorphenoxy)-2-ethoxy-3,3-dimethyl-1-(1,2,4--triazol-1-yl)-1-buten, 2-(4-Chlorbenzyloxy)-1-(4-chlorphenoxy)-3,3-dimethyl-1-(1,2,4-triazol-1-yl)-1-buten, 1-(4-Chlorphenoxy)-2-(4-chlorphenyl)-2-methoxy-1--(1,2,4-triazol-1-yl)-ethen, 2-(4-Chlorphenoxy)-1-(4-chlorphenyl)-2-(4-chlorphenyl)-1-(1,2,4-triazol-1-yl)-ethen, 1-(4-Chlorphenyl)-3-methoxy-4,4-dimethyl-2-(1,2,4--triazol-1-yl)-2-penten, 1-(4-Chlorphenyl)-3-ethoxy-4,4-dimethyl-2-(1,2,4--triazol-1-yl)-2-penten, 3-(4-Chlorbenzyloxy)-1-(4-Chlorphenyl)-4,4-dimethyl--2-(2,2,4-triazol-1-yl)-2-penten, 1-(2,4-Dichlorphenyl)-3-methoxy-4,4-dimethyl-2-(1,2,4--triazol-1-yl)-1-penten

5. Verfahren zur Herstellung der Entriazole der Formel I gemäß Anspruch 1, dadurch gekennzeichnet, daß man
a) ein Triazolylketon der Formel IV 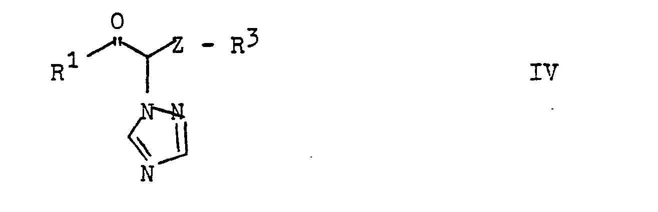 worin _{R}¹, R³ und Z die angegebene Bedeutung haben oder
b) - falls die Doppelbindung in Position b ist und Z ein Kohlenstoffatom bedeutet - eine Verbindung der Formel V 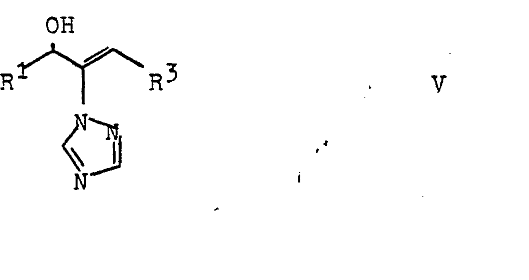 worin R¹ und R³ die angegebene Bedeutung haben, mit einer Verbindung der Formel VI 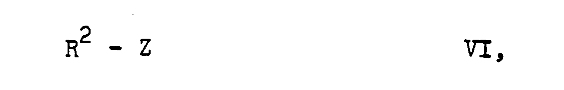 worin R² die angegebene Bedeutung hat und Z eine nucleofuge Abgangsgruppe darstellt, in Gegenwart eines Säurefängers umsetzt und die so erhaltenen Verbindungen gegebenenfalls in ihre Säureadditionssalze oder Metallkomplexe überführt.

6. Fungizides Mittel, enthaltend eine Verbindung gemäß Anspruch 1.

7. Fungizides Mittel, enthaltend eine Verbindung gemäß Anspruch 1 und einen festen oder flüssigen Trägerstoff.

8. Verfahren zur Bekämpfung von Pilzen, dadurch gekennzeichnet, daß man mindestens ein Entriazol der Formel I gemäß Anspruch 1 auf diese einwirken läßt.

9. Verfahren zur Herstellung von Fungiziden, dadurch gekennzeichnet, daß man mindestens ein Entriazol der Formel I gemäß Anspruch 1 mit festen oder flüssigen Trägerstoffen mischt.

10. Verfahren zur vorbeugenden Bekämpfung von Pilzen, dadurch gekennzeichnet, daß man mindestens ein Entriazol der Formel I gemäß Anspruch 1 auf durch Pilzbefall bedrohte Flächen, Pflanzen oder Saatgüter einwirken läßt.
